(19) Europäisches Patentamt
European Patent Office
Office européen des brevets

(11) Publication number: **0 274 009 B1**

(12) **EUROPEAN PATENT SPECIFICATION**

(45) Date of publication of patent specification: **09.09.92**

(51) Int. Cl.5: **C07D 271/06**, //C07D487/04, A61K31/505

(21) Application number: **87114729.4**

(22) Date of filing: **01.10.86**

(60) Publication number of the earlier application in accordance with Art.76 EPC: **0 226 282**

(54) **Oxadiazolylacetonitriles, their preparation and use.**

(30) Priority: **17.10.85 DK 4768/85**
**17.10.85 DK 4769/85**

(43) Date of publication of application:
**13.07.88 Bulletin 88/28**

(45) Publication of the grant of the patent:
**09.09.92 Bulletin 92/37**

(84) Designated Contracting States:
**AT BE CH DE ES FR GB GR IT LI LU NL SE**

(56) References cited:
**EP-A- 201 678**

**JOURNAL OF ORGANIC CHEMISTRY, vol. 41, no. 4, 20th February 1976, pages 620-626, Easton, PA, US; J.E. FRANZ et al.: "Periselective addition of nitrile sulfides, nitrile oxides, and diphenyldiazomethane to tetracyanoethylene"**

(73) Proprietor: **NOVO NORDISK A/S**
**Novo Allé**
**DK-2880 Bagsvaerd(DK)**

(72) Inventor: **Wätjen, Frank**
**Ravnehusvej 27**
**DK-3500 Vaerloese(DK)**
Inventor: **Engelstoft, Mogens**
**Mosegard Park 121**
**DK-3500 Vaerloese(DK)**

(74) Representative: **Perry, Robert Edward et al**
**GILL JENNINGS & EVERY 53-64 Chancery Lane**
**London WC2A 1HN(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention provides 3-substituted-5-isocyanomethyl-1,2,4-oxadiazoles of formula XI and 5-substituted-3-isocyanomethyl-1,2,4-oxadiazoles of formula XII. EP-A-0201678 discloses 5-cyclopropyl-3-isocyanomethyl-1,2,4-oxadiazole, but the novel compounds are the remainder of the given types in which the 5-substituent or 3-substituent, respectively is $C_{1-6}$ alkyl or $C_{3-6}$ cycloalkyl.

The novel compounds are useful as intermediates in the preparation of the therapeutically-active quinazolines described and claimed in EP-B-0226282 (corresponding to Application No. 86307541.2 from which this application has been divided).

The following description illustrates the preparation of particular examples of the invention.

5-(Ethyl or Methyl)-3-isocyanomethyl-1,2,4-oxadiazole

The following compounds were synthesised from the appropriate ethyl esters, as described below, in a manner similar to that described in EP-A-0201678, for 3-formylaminomethyl-5-cyclopropyl-1,2,4-oxadiazole:

3-Formylaminomethyl-5-ethyl-1,2,4-oxadiazole. H-NMR (60 MHz, $CDCl_3$) (ppm): 1.4 (3H, t, J = 8 Hz), 2.9 (2H, q, J = 8 Hz) 4.55 (2H, s), 7.8 (1H, broad-NH), 8.25 (1H, s).

3-Formylaminomethyl-5-methyl-1,2,4-oxadiazole. H-NMR (60 MHz, $CDCl_3$) (ppm): 2.6 (3H, s), 4.6 (2H, d, J = 3 Hz), 7.4 (1H, broad-NH), 8.25 (1H, s).

These two compounds were then converted, as described below in a manner similar to that described in EP-A-0201678 for 5-cyclopropyl-3-isocyanomethyl-1,2,4-oxadiazole which does not form part of the invention to 5-ethyl-3-isocyanomethyl-1,2,4-oxadiazole and 5-methyl-3-isocyanomethyl-1,2,4-oxadiazole. Both these were oils and were characterised by their IR stretching band at 2160 $cm^{-1}$.

3-cyclopropyl-5-isocyanomethyl-1,2,4-oxadiazole

a. 3-cyclopropyl-5-formylaminomethyl-1,2,4-oxadiazole.

A solution of ethyl formylaminomethyl-carboxylate (150 mmol) and cyclopropyl carboxamide oxime (100 mmol) in 100% EtOH (100 ml) was charged with Na (200 mg) and a crushed molecular sieve (4A) (10 g). The mixture thus obtained was stirred and heated to reflux for 8 hours. The mixture was cooled to room temperature, filtered through filter aid and the filtrate was evaporated in vacuo. The oily residue was partitionated into a $CHCl_3$ phase which was dried with $Na_2SO_4$ and evaporated.

b. 3-cyclopropyl-5-isocyanomethyl-1,2,4-oxadiazole.

A stirred solution of 3-cyclopropyl-5-formylaminomethyl-1,2,4-oxadiazole (60 mmol) and triethylamine (176 mmol) in $CH_2Cl_2$ (100 ml) was charged dropwise with $POCl_3$ (60 mmol) at 0 °C. The mixture was then left for 30 minutes with stirring at 0 °C, whereafter a solution of $Na_2CO_3$ (60 mmol) in $H_2O$ (50 ml) was added. The mixture was heated to room temperature, whereafter the organic phase was separated, dried and evaporated in vacuo. The residue was treated with ether, decanted and the solution was evaporated to give the title compound as an oil. The oil was processed without any further purification. The compound was characterized by its IR absorption band at 2160 $cm^{-1}$.

3-ethyl-5-isocyanomethyl-1,2,4-oxadiazole was prepared from 3-ethyl-5-formylaminomethyl-1,2,4-oxadiazole in a similar manner. IR: $cm^{-1}$: 2170.

5-cyclopropyl-3-isocyanomethyl-1,2,4-oxadiazol

a. Formylaminomethyl-carboxamide oxime.

0.55 mmol of freshly liberated hydroxylamine dissolved in 370 ml methanol was added to 53.6 g (0.638 mmol) N-formylamino-acetonitrile. An ice bath was used to keep the temperature below 20 °C during addition. The solution was allowed to stand at room temperature overnight, whereafter it was evaporated to give the title compound as pale crystals. Decomp. 104-110 °C.

b. 3-formylaminomethyl-5-cyclopropyl-1,2,4-oxadiazole

A mixture of 35 ml ethyl cyclopropylcarboxylate , 20 g formylamino-methylcarboxamide oxime, 1 g

sodium and 30 g of a crushed molecular sieve (4A) was refluxed in 300 ml abs. EtOH for 8 hours whereafter a further 1 g sodium was added The reaction mixture was filtered and the filtrate was evaporated. The dark oily residue was suspended in 300 ml $CHCl_3$, filtered and the filtrate was evaporated to give the title compound as an oil. H-NMR (60 MHz, $CDCl_3$) (ppm): 1.2 (4H, m), 2.8 (1H, m), 4.5 (2H, d, J=6Hz), 7.8 (1H, broad-NH), 8.2 (1H, s).

c. 5-Cyclopropyl-3-isocyanomethyl-1,2,4-oxadiazole

A stirred solution of 5-cyclopropyl-3-formylamino-methyl-1,2,4-oxadiazole(60 mmol) and triethylamine (176 mmol) in $CH_2Cl_2$(100 ml) was charged dropwise with $POCl_3$ (60 mmol) at 0°C. The mixture was then left for 30 minutes with stirring at 0°C, whereafter a solution of $Na_2CO_3$(60 mmol) in $H_2O$ (50 ml) was added. The mixture was heated to room temperature, whereafter the organic phase was separated, dried and evaporated in vacuo. The residue was treated with ether, decanted and the solution was evaporated to give the title compound as an oil. The oil was processed without any further purification. The compound was characterized by its IR absorbtion band at 2160 $cm^{-1}$.

**Claims**

**1.** A 5-isocyanomethyl-1,2,4-oxadiazole of formula XI

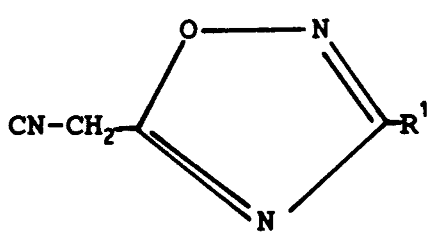

XI

wherein $R^1$ is $C_{1-6}$ alkyl or $C_{3-6}$ cycloalkyl.

**2.** A 3-isocyanomethyl-1,2,4-oxadiazole of formula XII

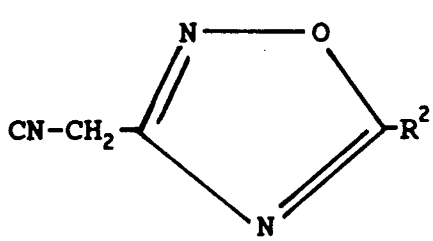

XII

wherein $R^2$ is $C_{1-6}$ alkyl or $C_{4-6}$ cycloalkyl.

**3.** A compound of claim 1, wherein $R^1$ is ethyl or cyclopropyl.

**4.** A compound of claim 2, wherein $R^2$ is methyl or ethyl.

**Patentansprüche**

**1.** 5-Isocyanomethyl-1,2,4-oxadiazol der Formel XI

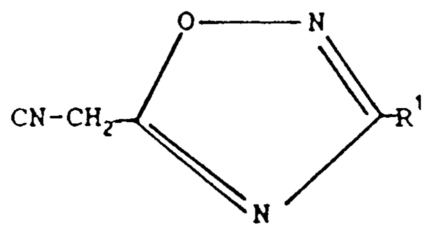

XI

worin $R^1$ $C_{1-6}$-Alkyl oder $C_{3-6}$-Cycloalkyl ist.

**2.** 3-Isocyanomethyl-1,2,4-oxadiazol der Formel XII

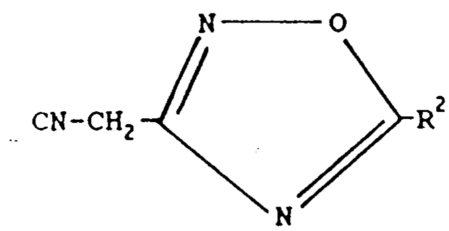

XII

worin $R^2$ $C_{1-6}$-Alkyl oder $C_{4-6}$-Cycloalkyl ist.

**3.** Verbindung nach Anspruch 1, worin $R^1$ Ethyl oder Cyclopropyl ist.

**4.** Verbindung nach Anspruch 2, worin $R^2$ Methyl oder Ethyl ist.

**Revendications**

**1.** 5-isocyanométhyl-1,2,4-oxadiazole de formule XI

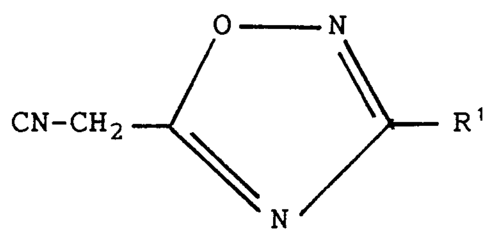

XI

dans laquelle $R^1$ est un alkyle en $C_{1-6}$ ou un cycloalkyle en $C_{3-6}$.

**2.** 3-isocyanométhyl-1,2,4-oxadiazole de formule XII

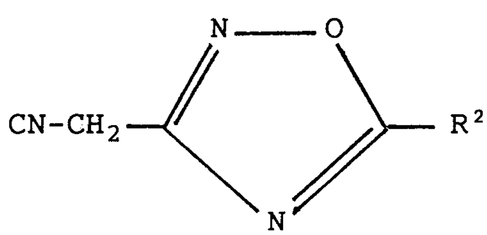

XII

dans laquelle $R^2$ est un alkyle en $C_{1-6}$ ou un cycloalkyle en $C_{4-6}$.

**3.** Composé de la revendication 1, dans lequel $R^1$ est éthyle ou cyclopropyle.

**4.** Composé de la revendication 2, dans lequel $R^2$ est méthyle ou éthyle.